# EUROPEAN PATENT SPECIFICATION

(11) **EP 0 130 633 B1**
(45) Date of publication and mention of the grant of the patent: **09.10.1996**
(21) Application number: 84200772.6
(22) Date of filing: 29.05.1984
(51) Int. Cl.: C07D 263/10, C07D 263/20, C07D 498/04

(54) **Process for the preparation of 1-(phenyl)-1-hydroxy-2-amino-3-fluoro propane derivatives**
Verfahren zur Herstellung von 1-(Phenyl)-1-hydroxy-2-amino-3-fluor-propan-Derivaten
Procédé de préparation de dérivés de 1-phényl-1-hydroxy-2-amino-3-fluoro propane

(30) Priority: 02.06.1983 IT 2141783; 05.08.1983 IT 2244983; 03.02.1984 IT 1943584
(43) Date of publication of application: 09.01.1985
(60) Divisional application: 95201522.0
(73) Proprietor: ZAMBON GROUP S.p.A., I-36100 Vicenza (IT)
(72) Inventor: Jommi, Giancarlo, I-20131 Milano (IT); Chiarino, Dario, I-20052 Monza (Milano) (IT); Pagliarin, Roberto, 20010 San Giorgio Su Legnano (Milano) (IT)

(56) References cited:
- EP-A- 0 014 437
- AT-B- 179 022
- US-A- 2 820 041
- Angewandte Chemie (International Edition), Vol. 20 (1981), pages 647 to 667
- Houben-Weyl, Methoden der Organischen Chemie, Vol. V/3 (1962), pages 166 to 169

## Description

This invention relates to a process for preparing 1-(phenyl)-1-hydroxy-2-amino-3-fluoro-propane derivatives of formula: 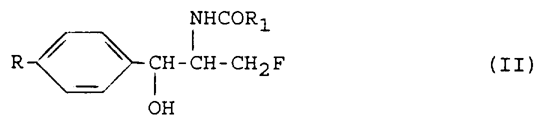 wherein
R is CH₃S-, CH₃SO- and CH₃SO₂; and
R₁ is dichloromethyl.

The compounds of formula II contain two asymmetric carbon atoms and can exist as stereoisomers. The D-(threo)-forms are preferred because of their broader antibacterial activity. U.S. Patent 4,235,892 discloses the compounds of formula II and a process for their preparation. This process essentially consists in N-protecting a 1-(phenyl)-2-amino-1,3-propanediol, (the phenyl moiety of which is variously substituted) by an imido derivative of a bicarboxylic acid, in treating the thus obtained compound with dialkylaminosulfotrifluoride (DAST), in removing the N-protecting group and then in acylating the thus obtained 1-(phenyl)-1-hydroxy-2-amino-3-fluoro-propane with the desired haloacetic acid or with a suitable reactive derivative thereof.

Although apparently easy, this process suffers many disadvantages and affords low yields.

One of the main disadvantages is that the fluorination of the primary hydroxy group is not selective and leads to the formation of many by-products, among which may be mentioned, for instance, the products deriving from the substitution at the secondary hydroxy group. The desired compound may thus be obtained at a sufficient purity degree only by a particularly complex column chromatography process. Another disadvantage is that DAST is the only agent which allows to perform the fluorination step on the peculiar intermediate products which are prepared according to the process of US Patent 4,235,892 and DAST is very expensive and dangerous, especially when it is intended for large scale production.

It has been now found that the above mentioned disadvantages can be overcome by using new compounds of formula I wherein either the amino group or the secondary hydroxy group of the 1-(phenyl)-2-amino-1,3-propanediol derivative are protected. Also the primary hydroxy group is substituted by a leaving group which can be replaced by a fluorine atom.

Thus, object of the present invention is a process for the preparation of the compounds of formula II substantially free from by-products deriving from replacement of the secondary hydroxy group with a fluorine atom or racemization of the asymmetric carbon atoms,
characterized in that
(i) both the secondary hydroxy and the amino group of a D-threo 1-(phenyl)-2-amino-1,3-propanediol are protected via a D-threo compound of the formula 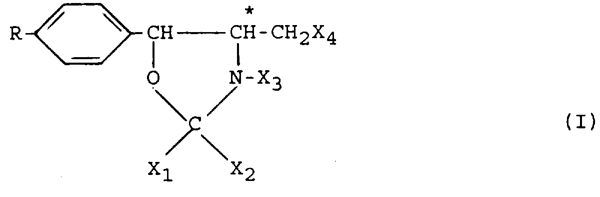 where
   R has the above mentioned meanings;
   X₁ is hydrogen, 1-6 C alkyl, 1-6 C haloalkyl, 3-6 C cycloalkyl, phenyl or phenylalkyl (1-6 C), where the phenyl ring may be substituted by one or two halogen, 1-3 C alkyl, 1-3 alkoxy or nitro; or
   X₁ together with X₂ is an oxygen atom or an alkylene having from two to five carbon atoms; or
   X₁ together with X₂ and R₄ is a chain of formula 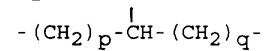 where p is 3 or 4, q is 1 or 2; and
   X₂ is hydrogen, 1-6 C alkyl, 1-6 C haloakyl, 3-6 C cycloalkyl or phenyl which may be substituted by one or two halogen, 1-3 C alkyl, 1-3 C alkoxy or nitro; or
   X₂ together with X₁ has the above mentioned meanings; or
   X₂ together with X₃ is a covalent bond; or
   X₂ together with R₄ is

      -(CH₂)ₘ-CH=CH-; -(CH₂)ₘ-CH₂-CH₂-

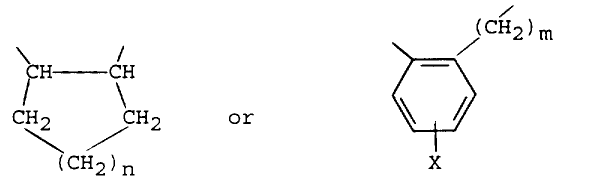

      where n is 1 or 2; m is 0 or 1; X is hydrogen, halogen, 1-3 C alkyl, 1-3 C alkoxy or nitro; or
   X₂ together with X₁ and R₄ has the above mentioned meanings; and
   X₃ is hydrogen or -CO-R₄ where R₄ is hydrogen, 1-6 alkyl, 1-6 C haloalkyl, 3-6 C cycloalkyl, phenyl or phenyl-alkyl(1-6C), where the phenyl ring may be substituted by one or two halogen, 1-3 C alkyl, 1-3 C alkoxy or nitro, or
   R₄ together with X₂ has the above mentioned meanings; or
   R₄ together with X₂ and X₁ has the above mentioned meanings; or
   X₃ together with X₂ has the above mentioned meanings; and
   X₄ is -O-SO₂R₆ where R₆ is methyl, trifluoromethyl, phenyl or p-methyl-phenyl; and
(ii) the D-threo compound of formula (I) is fluorinated with KF in a polyglycol to afford a D-threo compound of the formula 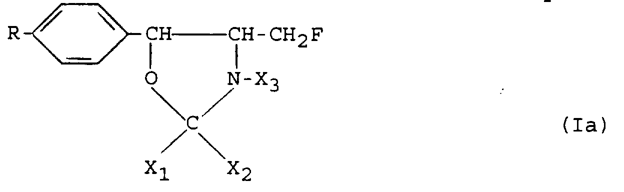 where R, X₁, X₂ and X₃ have the above mentioned meanings; and
(iii) the D-threo compound of formula (Ia) is hydrolyzed to afford a D-threo compound of the formula 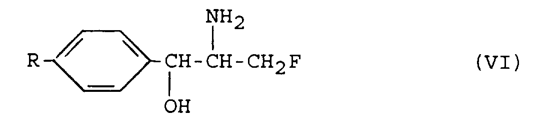 where R has the above mentioned meanings; and
(iv) the D-threo compound of formula (VI) is acylated with a compound of formula R₁COOH, wherein R₁ has the above mentioned meanings, or a reactive derivative thereof to afford the desired D-threo compound of formula (II).

The use of the compounds of formula I has the advantage to avoid the formation of many by-products and this not only in that the protection hinders the fluorination of the secondary hydroxy group but also in that it insures the stability of the configuration of the asymmetric carbon atoms.

Furthermore the compounds of formula I are quite soluble in the most part of the aprotic organic solvents, thus allowing to carry out the fluorination step also in homogeneous phase and under anhydrous and mild conditions.

Another advantage is that the compounds of formula I are prepared very easily by making use of cheap reactants and that the protective groups can be removed very easily as well after the completion of the fluorination step.

Still another advantage of the compounds of formula I is that the protection of the secondary hydroxy group allows to substitute the primary hydroxy group with a suitable leaving group so that the fluorination step can be most conveniently carried out with fluorination agents which are cheaper and less dangerous than DAST.

The protection of the amino and of the secondary hydroxy group thus performed is substantially inert to those treatments that the molecule undergoes, more particularly with nucleophiles and bases, up to the fluorination step and can then be easily removed under mild conditions.

When X₁, X₂ and R₄, equal or different among them, are hydrogen, 1-6 C alkyl, 1-6 C haloalkyl, 3-6 C cycloalkyl, phenyl or substituted phenyl, examples of suitable reactants are: aldehydes such as formaldehyde, acetaldehyde, valeraldehyde, caproaldehyde, benzaldehyde, anisaldehyde, 4-chlorobenzaldehyde, 4-ethoxy-3-methoxy-benzaldehyde, 2,6-dinitrobenzaldehyde or ketones such as acetone, diethylketone or hexylmethylketone for protecting the secondary hydroxy group and one hydrogen of the amino group, and acids such as acetic, dichloroacetic, trifluoroacetic, pivaloyl, benzoic, 2,4-dibromobenzoic, veratric, 2,5-dimethylbenzoic or 4-nitrobenzoic acid, for protecting the second hydrogen of the amino group.

When X₁ and X₂, together, are an alkylene radical having from 2 to 5 carbon atoms, examples of suitable reactants are the cycloalkanones such as cyclopropanone, cyclopentanone or cyclohexanone.

When X₁ together with X₂ and R₄ is a chain of formula 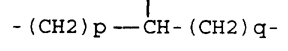 where p and q have the above mentioned meanings, examples of suitable reactants are the ketoacids such as (2-oxocyclopentyl) acetic acid, (2-oxocyclohexyl)-acetic acid, 3-(2-oxocyclopentyl) propionic acid and 3-(2-oxocyclohexyl)-propionic acid.

When X₂ together with R₄ forms a mono- or a poly-cylic system, examples of suitable reactants are the aldehydo-acids or the keto-acids such as phthalaldehydic acid, succinic semialdehyde, levulinic acid, 4-phenyl-4-oxo-butyric acid; hexahydrophthalaldehydic acid; (2-acetyl)-cyclohexylcarboxylic acid and (2-acetyl)-cyclopentyl-carboxylic acid.

When X₁ together with X₂ is an oxygen atom, examples of suitable reactants are the halocarbonates of formula XCOOR₂ where X is a halogen atom and R2 is an alkyl, aralkyl or an aryl radical; preferably R₂ is an 1-4 C alkyl radical.

Preferred compounds according to the process of this invention are those wherein X₂ and X₃ together are a covalent bond and X₁ is hydrogen, 1-6 C alkyl, 1-6 C haloalkyl, phenylalkyl(1-6C), phenyl where the phenyl ring may be substituted by one or two halogen, 1-3 C alkyl, 1-3 C alkoxy or nitro. Examples of suitable reactants for preparing such a compounds are the amidines of formula X₁-C(=NH)-NH₂ or their salts, the halohydrates of iminoethers of formula X₁-C-(OR')=NH₂⁺·X, the orthoesters of formula X₁-(OR')₃ and the nitriles of formula X₁-CN, where X₁ has the above mentioned meanings, X is halogen and R' is preferably an alkyl having low molecular weight. A preferred reactant is benzamidine.

The methods for preparing the compounds of formula I change according to the nature of the desired compound. Excepting those where X₁ and X₂ together are an oxygen atom, they can be prepared according to known techniques.

Examples of known techniques for preparing oxazolines, i.e. the compounds of formula I where X₂ and X₃, together, are a covalent bond, are disclosed by E.H. Rodd ("Chemistry of Carbon Compounds" vol. IV; Heterocyclic Compounds, page 361, Elsevier Publishing Co. 1957) and by R.C. Elderfield ("Heterocyclic Compounds", vol. V, page 377, J. Wiley and Sons, Inc., 1957) as well as by Angew. Chem. Int. Ed. 15, 270-281, 1976; Chem. Rev. 71, 483506, 1971; Chem. Rev. 44, 447-476, 1941.

As mentioned before, the methods for preparing oxazolines involve the reaction of a compound of formula 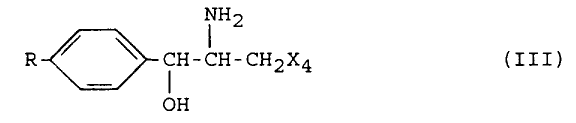 where R and X₄ have the above mentioned meanings, with a suitable reactant such as an amidine, a halohydrate of an iminoether, an orthoester or a nitrile.

Alternatively they are prepared by dehydratation, catalyzed by acids, of amides of formula 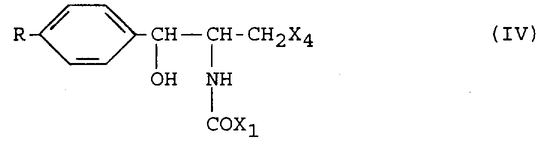 or by cyclization, catalyzed by bases, of active esters of formula 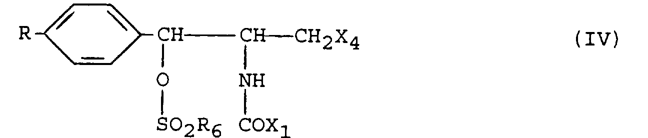 where X₁, X₄ and R₆ have the above mentioned meanings. When X₄ is -OH, the thus obtained oxazolines are then treated according to known techniques to afford the compounds where X₄ has the desired meaning.

The preparation of compounds I where X₁ and X₂, together, are an oxygen atom is based on the unexpected finding that compounds of formula 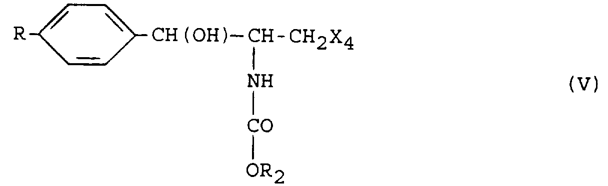 where R, X₄ and R₂ have the above mentioned meanings, cyclize regioselectively on the secondary hydroxy group to afford oxazolidinones of formula I, in the presence of strong bases and of aprotic solvents.

The role of the solvent is critical. When the reaction is carried out in the presence of a non-aprotic solvent, the cyclization proceeds either on the secondary or on the primary hydroxy group and affords a mixture of the two possible cyclic compounds.

Examples of suitable aprotic solvents are the aromatic hydrocarbons such as benzene and toluene. Examples of suitable strong bases are the alkali and the alkaline earth metal alcoholates as well as the tertiary amines.

The cyclization reaction may also proceed via the intermediate formation of the alcoholate at the secondary hydroxy group when they are used alkali and alkaline earth metal alcoholates, alkali metal hydrides such as sodium hydride, sodium amide, Grignard-like organo-metallic derivatives and alkyl-lithium derivatives.

In their turn, the compounds (V) can be prepared according to known techniques such as the reaction of the desired 1-(phenyl)-2-amino-1,3-propanediol, substituted at the phenyl ring, with a compound of formula X-COOR₂, where X is halogen and R₂ has the above mentioned meanings, in the presence of a base and of a suitable diluent.

When it is used an organic diluent such as acetonitrile, it is preferably used an organic base such as a tertiary amine whereas is preferably used an inorganic base such as an alkali metal carbonate or bicarbonate, when the reaction is carried out in aqueous medium. Alternatively it may be used a basic diluent such as pyridine.

The compounds of formula V wherein R is -SCH₃ are more soluble into the aprotic solvents, than those wherein R is -SO-CH₃ or SO₂CH₃. A preferred way for preparing compounds (I) wherein R is -SO-CH₃ or -SO₂CH₃ comprises the preparation of the corresponding compounds (V) wherein R is -S-CH₃, their subsequent cyclization and lastly, their oxidation according to known techniques.

The substitution of the hydrogen of the primary hydroxy group with a -SO₂R₆ radical, where R₆ has the above mentioned meanings, can be carried out before or after the protection of both the secondary hydroxy and amino group. Also this substitution can be carried out according to known techniques.

Examples of desired fluorination agents when X₄ is -O-SO₂-R₆, where R₆ has the above mentioned meanings, are the alkali and the alkaline-earth metal fluorides.

Anyway, the replacement of the group X4 with fluorine is a complex technical problem particularly when it is desired to use the most economic and amenable fluorination agents, that is the alkali and alkaline-earth metal fluorides, because F anion behaves besides as a nucleophilic agent also as a base causing competitive side-reactions such as elimination, hydrolysis or, whenever it is possible, solvolysis (E.V. & S.M. Dehmlow "Phase transfer catalysis" Verlag Chemie, 1980, page 80). Because of the peculiar nature of the hydrogen atom that is marked with an asterisk in formula (I), the compounds (I) undergo elimination. When they have been reacted with F^{⊖} in phase transfer conditions or in the presence of crown ethers, they did not react or did afford low yields of the desired compound owing to the concomitant formation of hydrolization and elimination products.

Now, it has been found that these drawbacks can be overcome when the reaction between a compound (I) wherein X₄ is -O-SO₂₋R6, where R₆ has the above mentioned meanings, and F^{⊖} is carried out in the presence of a suitable polyglycol. Preferably, the fluorination step will be carried out according to the last technique.

After performance of the fluorination step, the protective groups are removed from the compounds of formula I wherein X₄ is fluorine.

A preferred method is consisting in removing the protective groups with acids, preferably inorganic acids, in aqueous medium or in water/organic diluents mixtures. The latter media are preferred when hydrolysis regenerates the compound or the compounds which had been previously used as protective agents and when the amine which is formed is soluble in an aqueous solution of inorganic acids. It is thus obtained a repartion of the amine in the aqueous layer and of the protective agent or agents in the organic layer, from which they are recovered and then recycled; in its turn, the amine is recovered by precipitation via neutralization of the aqueous layer. Alternatively the amine can be extracted with a suitable organic solvent.

When X₁ and X₂, together are an oxygen atom, the protective group may also be removed through treatment with an organo-metallic derivative such as a Grignard's derivative and the subsequent hydrolysis in mild conditions with inorganic acids in water or in water/organic solvent mixtures.

Another possible method for removing the protective group when X₁ and X₂, together, are an oxygen atom, comprises the reduction of the keto group and the subsequent hydrolysis in mild conditions as described above. This reduction is preferably carried out with complex hydrides such as sodium borohydride. After removal of the protective groups it is obtained a compound of formula 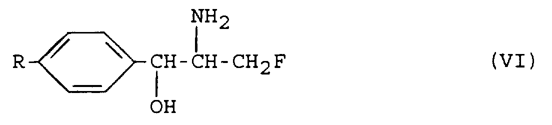 which can be reacted with a haloacetic acid of formula R₁COOH, wherein R₁ has the above mentioned meanings, or a reactive derivative thereof to afford the desired compound of formula (II).

The protection performed by the compounds of formula I is useful not only for preparing the compounds of formula II, but also whenever it is desired to replace the primary hydroxy group with another functional group, such as chlorine, bromine, iodine, nitrile, hydrogen, -OR₇, -SR₇, -SCOR₇, -SCN, -S(=NH)NH₂, -CH-(COOR₇)₂, wherein R₇ is alkyl, aralkyl or aryl. This invention is illustrated by the following examples which should not be constructed as limiting it in any way.

### EXAMPLE 1

### Preparation of 3-acetoxy-1-(4-methylsulfonyl-phenyl)-2-phthalimido-1-hydroxy-propane (A).

D-(threo)-1-(4-methylsulfonylphenyl)-2-phthalimido-1,3-propanediol (1 g; 2.66 mmols), prepared as described in U.S. Patent 4,235,892, has been dissolved in anhydrous pyridine (5 ml). Acetylchloride (0.2 ml; 2.83 mmols) has been added dropwise to this solution kept under stirring at O°C; after completion of the addition, the reaction mixture has been heated to 25°C and kept under stirring for 1 hour; afterwards, the reaction mixture has been poured into water and ice, acidified with hydrochloric acid and extracted with ethyl acetate.

The crude product (A) has been obtained (quantitative yield) from the organic layer after drying over sodium sulfate and evaporation of the solvent in vacuo; the crude, after crystallization from methanol, gave a pure product (0.84 g; yield 75%) as shown by HPLC and TLC analysis.

| Elemental Analysis for C₂₀H₁₉O₉N | | | |
|---|---|---|---|
| (found) | C 57.3 %; | H 4.6%; | N 3.3 % |
| (Calculated) | C 57.55 %; | H 4.56 %; | N 3.36 %. |

The acetylation is regio-selective on the secondary hydroxy group as shown by NMR spectrum in DMSO; delta=1.78; s, 3H, CH₃CO-; 4.50 dd- 2H, -CH₂OAc; 6.02,d,1H, benzylic OH.

### EXAMPLE 2

Reduction of compound (A) to 3-acetoxy-1-(4-methylsulfonylphenyl)-1-hydroxy-2-(3-hydroxy-1H-isoindol-1-one-2-yl)-propane (B).

Compound (A) (0.76 g; 1.82 mmols) has been added to a mixture of tetrahydrofuran and water (1:1; 4 ml); to this suspension, kept at 0°C under vigorous stirring, has been added portionwise sodium borohydride (138 mg; 3.64 mmols). As the reaction proceeded, the suspension became a homogeneous solution, after 1 hour and after having checked by TLC the disappearance of compound (A) and the formation of a new product, tetrahydrofuran has been evaporated in vacuo and the product extracted with ethyl acetate.

After drying over sodium sulfate and evaporation of the solvent, compound (B) (0.7 g; yield 92%) has been obtained sufficiently pure to undergo as such the following reaction (Example 3).

Compound (B) proved to be a mixture of two diastereoisomers because of the formation, during the reduction step, of a new asymmetric carbon atom; this has been proved by TLC, HPLC and NMR spectra in DMSO containing D₂O; delta 1.78; s, and 1.86, s, 3H on the whole, CH₃-CO in two diastereoisomers in the ratio 35:65; 5.84,s, and 6.24,s,1H, on the whole 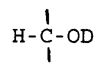 of the isoindole system (doublets, before deuteration, coupled with two doublets exhibiting delta = 6.8 and 6.57 respectively, 1H on the whole for -OH in the two diastereoisomers) and, finally, 5.14, d, and 5.2, d, 1H on the whole for the two 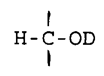 in position 1 of the propane chain.

### EXAMPLE 3

### Cyclization of compound (B) to 3-acetoxymethyl-2-(4-methylsulfonyl-phenyl)-2,3-dihydro-oxazole-[2,3,a]-isoindol-5(9bH)-one (C).

Product (B) (0.55 g; 1.3 mmol) has been suspended in benzene (5 ml) containing a little amount of p-toluensulfonic acid (5 mg); by heating, the mixture became a clear solution. A short time later the water formed during the reaction has been distilled azeotropically until water was absent in the distilled benzene and TLC analysis showed the disappearance of product (B). At the end, almost all benzene has been evaporated in vacuo; after having added some water, product (C) has been extracted with ethyl acetate. Crude product (C) has been obtained in quantitative yield from the organic phase after drying over sodium sulfate and evaporation of the solvent in vacuo.

Crude product (C) has been used as such for the subsequent hydrolysis (Example 4). An aliquot has been purified by chromatography on silica gel using ethyl acetate/petroleum ether in various ratios or pure ethyl acetate as eluents. It has been proved that the crude contained small amounts of some unidentified impurities; after purification by chromatography has been proved to be a mixture of two diastereoisomers as shown by TLC and HPLC analysis as well as by NMR spectrum in DMSO: delta 6.38,s, and 6.07 s, 1H on the whole, 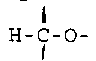 of the isoindole system; 2.08 s and 2.18 s, 3H on the whole, CH₃CO-; 3.19, s and 3.24, s, 3H on the whole, CH₃SO₂-.

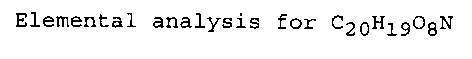

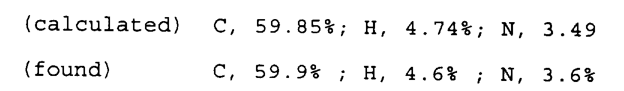

### EXAMPLE 4

### Hydrolysis of compound (C) to 2-(4-methylsulfonyl-phenyl)-3-hydroxy-methyl-2,3-dihydro-oxazole-[2,3,a]-isoindol-5(9bH)-one (D).

Product (C) (0.2 g; 0.5 mmols) has been dissolved in methanol (2 ml) containing potassium hydroxide (42 mg; 0.75 mmols) at 0°C and under vigorous stirring. After 30' the hydrolysis has been checked by TLC and showed the disappearance of product (C).

Methanol has been evaporated in vacuo and in the cold; the residue has been treated with water and extracted with ethyl acetate. The organic layer has been dried and evaporated to afford product (D) which has been recrystallized from ethyl acetate (0.16 g; yield 89%). The presence of two diastereoisomers in product (D) has been shown by NMR spectrum in DMSO, delta = 6.3, s and 5.82,s, 1H on the whole, 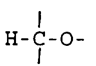 of the isoindole system; 3.18,s, and 3.22,s, 3H on the whole, CH₃SO₂-.

### EXAMPLE 5

### Preparation of 1,3-dihydroxy-1-(4-methylthio-phenyl)-2-ethoxycarbonylamino-propane (H).

D-(threo)-1-(4-methylthio)-phenyl-2-amino-1,3-propanediol (1.06 g; 4.93 mmols) has been suspended into an aqueous solution of potassium carbonate (1.8 g of potassium carbonate into 20 ml of water) and the thus obtained mixture has been cooled, under vigorous stirring, to 0°C.

Ethyl chlorocarbonate (0.5 ml) has been dropped quickly into the mixture maintained under vigorous stirring at 0°C; after half a hour, further 0.24 ml of ethyl chlorocarbonate (total amount: 7.74 mmols) has been added and the mixture has been maintained under stirring for 1 further hour.

At first the reaction mixture became clear and then a white precipitate has been slowly formed. After having checked the completion of the reaction by TLC, the suspension has been extracted with ethyl acetate. After drying on sodium sulfate, filtration and evaporation of the solvent, the organic extracts afforded 1.37 g of crude (H) (yield, 95.5%) which has been recrystallized from ethyl acetate/diisopropyl ether. m.p. = 75°C.
I.R. spectrum: 3340 and 3450 cm⁻¹ (OH, NH stretching), 1690-1700 cm⁻¹ (broad band: C=O amide).

In a similar manner it has been prepared the 1,3-dihydroxy-1-(4-methylsulfonyl-phenyl)-2-ethoxycarbonylamino-propane (I) which, after crystallization from ethyl acetate, showed (IR analysis) the following peaks: 3200-3360 cm (broad band - OH and NH stretching), 1715 cm (CO amide).

### EXAMPLE 6

### Cyclization of compound (H) to 5-(4-methylthio-phenyl)-4-hydroxy-oxazolidin-2-one (J).

Compound (H) (5 g; 17.5 mmols) has been dissolved in warm toluene (25 ml). To this solution, an equimolar amount of potassium ter.butylate has been added and the reaction mixture has been refluxed for 3 hours. Afterwards, almost all the solvent has been evaporated; water and ice have been added to the residue and the precipitate has been collected by filtration. The thus obtained crude (J) has been recrystallized from ethanol (3.7 g; yield, 88%); m.p. 130-131°C.
I.R. Spectrum: 3180, 3240, 3300 cm⁻¹ (OH and NH stretching) 1720; 1745 cm⁻¹ (C=O, oxazolidinone);

NMR in DMSO, delta: 7.64 and 8.0, two doublets, 2H each one of p-substituted phenyl; 7.88, S, 1H, NH amido; 5.48, d, 1H, benzyl hydrogen; 3.56, m, 2H, hydroxymethyl; 5.16, m, H linked to C4 of oxazolidinone ring; 3.2, S, 3H, CH₃S-.

### EXAMPLE 7

### Oxidation of compound (J) to 5-(4-methylsulfonyl-phenyl)-4-hydroxymethyl-oxazolidin-2-one (K).

Compound (J) (53 g; 221 mmols) has been added portionwise to 84 ml of hydrogen peroxide (130 vol.) maintained under stirring at 40-45°C. After completion of the addition, the stirring has been continued for further 20 hours at 40°C.

Acetic anhydride (76.6 g; 20.7 ml) has been dropped into the reaction mixture by keeping the temperature below 40°C. The reaction mixture has been then cooled to 20-22°C and maintained under stirring at this temperature for 3 hours and, finally, allowed to stand in refrigerator overnight.

Afterwards, the solvent has been evaporated with caution in vacuo at 40°C; hot ethanol has been added to the thus obtained residue. The solvent has been again evaporated and the residue crystallized from methyl alcohol. 48.6 g of product (K) yield, 81%, m.p. 172-174°C.
I.R. spectrum: 1710 cm⁻¹ (C=O, amide) 3470, 3340, 3250, 3200 cm⁻¹ (OH and NH).

### EXAMPLE 8

### Preparation of 5-(4-methylsulfonyl-phenyl)-4-methane-sulfonyloxy-methyl-oxazolidin-2-one (M) from (K).

Compound (K) (200 mg; 0.84 mmols) has been dissolved in anhydrous pyridine (3 ml). The thus obtained solution has been cooled to 0°C and added with freshly distilled methanesulfonyl chloride (0.06 ml). The solution has been maintained at 0°C overnight and then diluted with an aqueous solution of hydrochloric acid (stoichiometric amount with respect to pyridine) and ice. After extraction with ethyl acetate, the organic extract has been dried over sodium sulfate and evaporated in vacuo to afford crude (M); yield, 85-90%.
I.R. spectrum: 3300 cm⁻¹ (NH), 1760 and 1740 cm⁻¹.

### EXAMPLE 9

### Preparation of D(-)-threo-2-phenyl-4-hydroxymethyl-5-(4-methylthiophenyl)-2-oxazoline (N).

i)D-(-)-threo-1-(4-methylthiophenyl)-2-amino-1,3-propane diol (20 g; 94 mmols) has been thoroughly mixed with benzamidine hydrochloride dihydrate (25 g; 130 mmols) and melted at 100°C. The melted mass, after 1 hour at said temperature has been cooled.
   To the cooled mass has been added ethyl alcohol (850 ml) and the mixture has been refluxed till complete dissolution. The solution has been then filtered with active carbon and cooled to -10°C. The crystalline precipitate has been collected by filtration and dried at 50°C under reduced pressure for 10 hours.
   19 g (69%) of compound (N) have been thus obtained, m.p. 174-176°C.
   The thus obtained product has been purified by crystallization from ethyl alcohol (400 ml), filtration and drying at 60 °C under reduced pressure for 8 hours.
   Yield, 17 g (62%); m.p. 175-177°C.
ii) A solution of 390.6 g (1.83 moles) of D-(-)-threo-1-(4-methylthiophenyl)-2-amino-1,3-propanediol and 374 g (2.01 moles) of ethyl benzimidate hydrochloride in 500 ml of water was warmed under stirring at 30°C for 20 hours. The mixture was cooled at 5°C and the resulting precipitate was filtered, washed with water and dried in vacuum at 50°C.
   The crude oxazoline was purified by recrystallization from ethanol to give 395 g (72%) of product (N), m.p. 172-73°C.

In a similar manner may be prepared from D-(-)-threo-1-(4-methylthiophenyl)-2-amino-1,3-propanediol and the proper imidate the following derivatives:
D-(-)-threo-2-(4-nitrophenyl)-4-hydroxymethyl-5-(4-methylthio phenyl)-2-oxazoline (O)
D-(-)-threo-2-benzyl-4-hydroxymethyl-5-(4-methylthiophenyl)-2-oxazoline (P).

### EXAMPLE 10

### Preparation of D-(-)threo-2-methyl-4-hydroxymethyl-5-(4-methylthiophenyl)-2-oxazoline (Q).

Ethyl acetoimidate hydrochloride (50 g; 400 mmols) has been suspended in dichloromethane (360 ml). To the thus obtained suspension has been added D-(-)-threo-1-(4-methylthiophenyl)-2amino-1,3-propanediol (76.37 g; 358 mmols). The reaction mixture has been stirred for 5 hours and then poured into ice and water (230 g).

The aqueous layer has been separated and extracted with dichloromethane (60 ml). The combined organic extracts have been washed with water, dried on sodium sulfate and evaporated to dryness.

Crystalline product (Q) (69.50 g; 81%) has been thus obtained which has been purified by crystallization from methylter.butyl-ether (1750 ml), filtration and drying in vacuo at 40-50°C. Yield, 55.60 g (65%) m.p. 111-113°C.

In a similar manner has been prepared D-(-)-threo-2-methyl-4-hydroxymethyl-5-(4-methylsulfonylphenyl)-2-oxazoline (R) melting at 153-155°C.

### EXAMPLE 11

### Preparation of D-(-)-threo-2-phenyl-4-hydroxymethyl-5-(4-methylsulfonylphenyl)-2-oxazoline (U).

D-(-)-threo-2-phenyl-4-hydroxymethyl-5-(4-methylthiophenyl)-2-oxazoline (5 g; 16.7 mmols) prepared as disclosed by Example 9 above, has been added portionwise to acetic anhydride (5.80 ml) under stirring at 35°C. Into the thus obtained suspension, has been dropped hydrogen peroxide (20 ml; 130 volumes) maintaining the temperature at 35-40°C by external cooling.

After completion of the addition, stirring has been continued at 35°C till complete dissolution and the solution is allowed to stand overnight at 4°C. To the reaction mixture has been added water (25 ml). The precipitate has been separated by filtration, washed on the filter till neutral and then dried in vacuo at 60°C to afford 4.4 g of the desired product (U) which has been purified by crystallization from methyl alcohol (400 ml).
Yield, 3.80 g (68.5%); m.p. 209-211°C.

### EXAMPLE 12

Preparation of D-(-)threo-2-dichloromethyl-4-methanesulfonyloxymethyl-5-(4-methylsulfonylphenyl)-2-oxazoline (V). Methanesulfonyl chloride (8.13 ml; 105 mmols) has been dropped in 15 minutes into a solution of D-(-)-threo-1-(4-methylsulfonylphenyl)-2-dichloroacetylamino-1,3-propane diol (35.60 g; 100 mmols) in anhydrous pyridine (136 ml) maintained under stirring at 0°C.

The reaction mixture has been kept under stirring for 1 further hour and then allowed to stand in refrigerator overnight. The thus obtained mixture has been poured into a mixture of concentrate hydrochloric acid (150 ml) and ice and extracted with ethyl acetate (500 ml x 2).

The combined organic extracts have been washed with 5% hydrochloric acid (200 ml) and then with water till neutral, dried over sodium sulfate and evaporated to dryness.

The residue has been purified by crystallization from ethyl alcohol (200 ml).

24.4 g (56%) of D-(-)-threo-1-(4-methylsulfonylphenyl)-2-dichloroacetylamino-3-methanesulfonyloxypropanol melting at 109-112°C have been thus obtained.

A mixture of this product (2 g; 4.6 mmols) and of methane sulfonic acid (0.1 ml) in 35 ml of 1,2-dichloroethane, has been refluxed in a round-bottomed flask equipped with a water trap for 3 hrs. The reaction mixture has been cooled and washed with water.

The organic layer has been separated, dried over sodium sulfate and evaporated to dryness. The residue (0.7 g) has been purified by chromatography on a silica gel column (70 g) eluting with dichloromethane/methyl alcohol (9.5:0.5). The fraction containing the desired product (V) has been isolated and evaporated to dryness.

It has been thus obtained a product (200 mg) melting at 116-118°C which show a NMR spectrum consistent with the structural formula.

In a similar manner may be prepared from D-threo-(1-(4-methylsulfonylphenyl)-2-trifluoroacetylamino-1,3-propanediol (British Patent No. 1534387) the correspondent D-(threo-(-)-2-trifluoromethyl-4-hydroxymethyl-5-(4-methylsulfonylphenyl)-2-oxazoline (W).

### EXAMPLE 13

### Preparation of D-(-)-threo-2-phenyl-4-methanesulfonyloxymethyl-5-(4-methylthiophenyl)-2-oxazoline (X).

A mixture of product (N) (4 g; 13.37 mmols) prepared as disclosed in Example 9 above, in anhydrous pyridine (15 ml) has been kept under stirring at room temperature for about 1 hr. After cooling to 0°C has been added dropwise methane sulfonyl chloride (1145 ml; 14.7 mmols) and stirring has been continued for 2 further hrs maintaining the temperature at 0°C. The mixture has been then allowed to stand at about 3°C for 3 hrs. Ice has been added to the reaction mixture and the thus obtained solid has been filtered and dried in vacuo at room temperature. 4.96 g of chromatographically (T.L.C.) pure product (T) have been thus obtained.
Yield, 98%; m.p. 107-108°C.

In a similar manner may be prepared the following compounds:
D-(-)-threo-2-methyl-4-methanesulfonyloxymethyl-5-(4-methylthiophenyl)-2-oxazoline (Y)
D-(-)-threo-2-benzyl-4-methanesulfonyloxymethyl-5-(4-methylthiophenyl)-2-oxazoline (Z)
D-(-)-threo-2-trifluoromethyl-4-methanesulfonyloxymethyl-5-(4-methylsulfonylphenyl)-2-oxazoline (a)
D-(-)-threo-2-(4-nitrophenyl)-4-methanesulfonyloxymethyl-5-(4-methylthiophenyl)-2-oxazoline (b).

### EXAMPLE 14

### Preparation of D-(-)-threo-2-phenyl-4-fluoromethyl-5-(4-methylthiophenyl)-2-oxazoline (c).

Product (X) (4 g; 10.61 mmols) prepared as disclosed in Example 13 and anhydrous potassium fluoride (6.164 g; 106 mmols) in polyethylene glycol 400 (25 ml) have been maintained under stirring at 100-105°C for 19 hrs. After having added water, the mixture has been extracted with ethyl ether (3 x 10 ml). The combined organic extracts have been dried over sodium sulfate and evaporated to dryness. The oily residue has been purified by flash chromatography on a silica gel column eluting at first with 900 ml of ethyl acetate/petroleum ether 1:9 and then with 1500 ml of ethyl acetate/petroleum ether 2:8.

2.19 (yield, 68.5%) of product (c) have been thus obtained. An analytically pure sample has been prepared by crystallization from isopropanol; m.p. 70-72°C.
NMR (CDCl₃) delta: 5.5 d, (J=7Hz) (H-C5); 5.08, d.d, (1H) (CH₂F); 4.13-4.71, m, 2H (H-C₄ and -CH₂F).

### EXAMPLE 15

### Preparation of D-(-)-threo-1-(4-methylthiophenyl)-2-amino-3-fluoro-1-propanol (d).

Product (c) (1.01 g; 3.35 mmols) prepared as disclosed in Example 14 has been added to 2N hydrochloric acid (18 ml) and maintained under stirring at 100-105°C. After 50 minutes, further 20 ml of 2N hydrochloric acid have been added and the stirring is continued at 100-105°C. After 3 hrs and a half the solution has been cooled and extracted with ethyl ether (20 ml x 2). The aqueous layer has been at first made basic with sodium bicarbonate and the saturated with potassium carbonate. Finally it has been extracted with chloroform (20 ml x 3). The combined organic extracts have been evaporated to dryness in vacuo. 0.64 g of product (d) have been thus obtained. Yield, 88%.

### EXAMPLE 16

### Preparation of D-(-)-threo-2-phenyl-4-methanesulfonyloxymethyl-5-(4-methylsulfonylphenyl)-2-oxazoline (e).

To a mixture of product (U) (0.3 g; 0.9 mmols) prepared as disclosed in Example 11, in pyridine (5 ml) has been added methanesulfonyl chloride (0.077 ml; 0.99 mmols) at 0°C. After having continued the stirring at 0°C for 10 minutes, the mixture has been allowed to stand in refrigerator overnight. The mixture has been then made acid, extracted with ethyl acetate (15 ml x 2), dried over sodium sulfate and evaporated to dryness in vacuo.
0.32 g of product (e) have been thus obtained. Yield, 87%.

### EXAMPLE 17

### Preparation of D-(-)-threo-2-phenyl-4-fluoromethyl-5-(4-methylsulfonylphenyl)-2-oxazoline (f).

Product (e) (0.32 g; 0.78 mmols) prepared as disclosed in Example 16 above and anhydrous potassium fluoride (0.53 g; 0.91 mmols) in polyethylene glycol (400 ml) have been kept under stirring at 90-95°C for 3 hrs, then at 75°C for 17 hrs and, finally, at 110°C for 6 hrs. The reaction mixture has been worked up as disclosed in Example 14 and have been thus obtained 0.13 g of product (f). Yield, 50%.
NMR (CDCl₃) delta: 5.76 d, (J=7Hz, H-C); 5.16, d.d, (1H) (-CH₂F); 4.2-4.75, m, (2H), (H-C₄ and -CH₂F).

## Claims

1. A process for preparing a D-threo compound of the formula 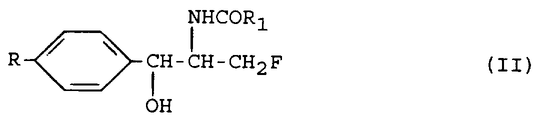 where
R is a methylthio, methylsulfoxy, methylsulfonyl; and
R₁ is dichloromethyl;
substantially free from by-products deriving from replacement of the secondary hydroxy group with a fluorine atom or racemization of the asymmetric carbon atoms,
characterized in that
(i) both the secondary hydroxy and the amino group of a D-threo 1-(phenyl)-2-amino-1,3-propanediol are protected via a D-threo compound of the formula 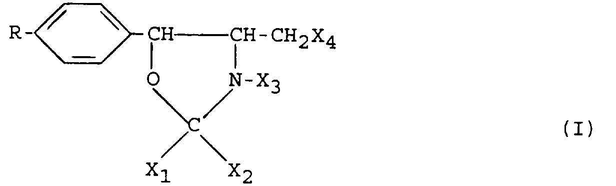 where
R has the above mentioned meanings;
X₁ is hydrogen, 1-6 C alkyl, 1-6 C haloalkyl, 3-6 C cycloalkyl, phenyl or phenylalkyl (1-6 C), where the phenyl ring may be substituted by one or two halogen, 1-3 C alkyl, 1-3 alkoxy or nitro; or
X₁ together with X₂ is an oxygen atom or an alkylene having from two to five carbon atoms; or
X₁ together with X₂ and R₄ is a chain of formula 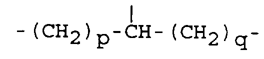 where p is 3 or 4, q is 1 or 2; and
X₂ is hydrogen, 1-6 C alkyl, 1-6 C haloakyl, 3-6 C cycloalkyl or phenyl which may be substituted by one or two halogen, 1-3 C alkyl, 1-3 C alkoxy or nitro; or
X₂ together with X₁ has the above mentioned meanings; or
X₂ together with X₃ is a covalent bond; or
X₂ together with R₄ is
-(CH₂)ₘ-CH=CH-; -(CH₂)ₘ-CH₂-CH₂- 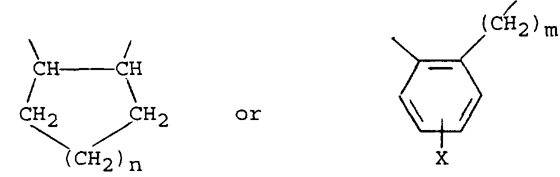
where n is 1 or 2; m is 0 or 1; X is hydrogen, halogen, 1-3 C alkyl, 1-3 C alkoxy or nitro; or
X₂ together with X₁ and R₄ has the above mentioned meanings; and
X₃ is hydrogen or -CO-R₄ where R₄ is hydrogen, 1-6 alkyl, 1-6 C haloalkyl, 3-6 C cycloalkyl, phenyl or phenyl-alkyl(1-6C), where the phenyl ring may be substituted by one or two halogen, 1-3 C alkyl, 1-3 C alkoxy or nitro, or R₄ together with X₂ has the above mentioned meanings; or R₄ together with X₂ and X₁ has the above mentioned meanings; or
X₃ together with X₂ has the above mentioned meanings; and
X₄ is -O-SO₂R₆ where R₆ is methyl, trifluoromethyl, phenyl or p-methyl-phenyl; and
ii) the D-threo compound of formula (I) is fluorinated with KF in a polyglycol to afford a D-threo compound of the formula 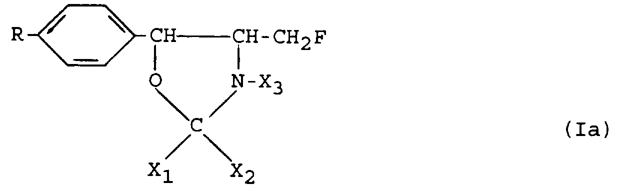 where R, X₁, X₂ and X₃ have the above mentioned meanings; and
(iii) the D-threo compound of formula (Ia) is hydrolyzed to afford a D-threo compound of the formula 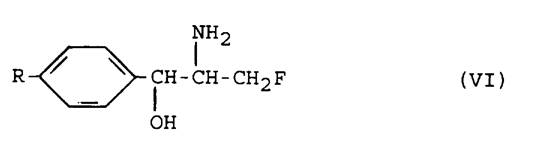 where R has the above mentioned meanings; and
(iv) the D-threo compound of formula (VI) is acylated with a compound of formula R₁COOH, wherein R₁ has the above mentioned meanings, or a reactive derivative thereof to afford the desired D-threo compound of formula (II).

## Patentansprüche

1. Verfahren zur Herstellung einer D-threo-Verbindung der Formel 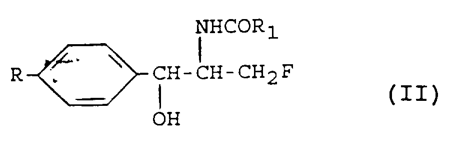 worin
R ein Methylthio, Methylsulfoxy, Methylsulfonyl bedeutet und
R₁ Dichlormethyl ist;
im wesentlichen frei von Nebenprodukten, die auf einem Austausch der sekundären Hydroxygruppe durch ein Fluoratom oder der Racemisierung der asymmetrischen Kohlenstoffatome basieren,
dadurch gekennzeichnet, daß
(i) sowohl die sekundäre Hydroxy- als auch die Aminogruppe eines D-threo-1-(Phenyl)-2-amino-1,3-propandiols als eine D-threo-Verbindung der Formel 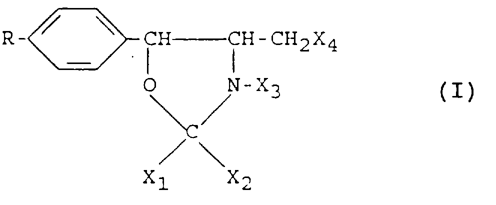 geschützt sind, worin
R die oben definierten Bedeutungen hat,
X₁ Wasserstoff, C₁₋₆-Alkyl, C₁₋₆-Halogenalkyl,
C₃₋₆-Cycloalkyl, Phenyl oder Phenylalkyl (C₁₋₆) ist, wobei der Phenylring substituiert sein kann durch ein oder zwei: Halogen, C₁₋₃-Alkyl, C₁₋₃-Alkoxy oder Nitro, oder
X₁ zusammen mit X₂ ein Sauerstoffatom oder ein Alkylen mit 2 bis 5 Kohlenstoffatomen bedeuten oder
X₁ zusammen mit X₂ und R₄ eine Kette der Formel 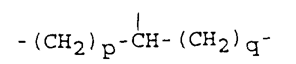 bedeuten, worin p = 3 oder 4,
q = 1 oder 2 ist und
X₂ Wasserstoff, C₁₋₆-Alkyl, C₁₋₆-Halogenaokyl, C₃₋₆-Cycloalkyl oder Phenyl ist, das substituiert sein kann durch ein oder zwei: Halogen, C₁₋₃-Alkyl, C₁₋₃-Alkoxy oder Nitro oder
X₂ zusammen mit X₁ die oben definierten Bedeutungen haben oder
X₂ zusammen mit X₃ eine kovalente Bindung darstellt oder X₂ zusammen mit R₄
- (CH₂)ₘ-CH=CH-; -(CH₂)ₘ-CH₂-CH₂- 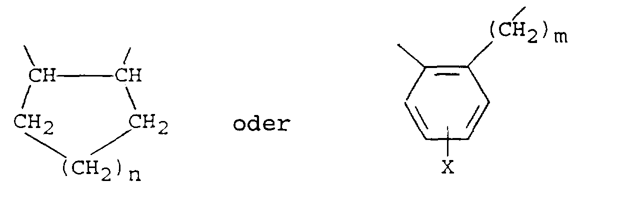
ist, worin n = 1 oder 2 bedeutet, m = 0 oder 1 ist,
X = Wasserstoff, Halogen, C₁₋₃-Alkyl, C₁₋₃Alkoxy oder Nitro ist oder
X₂ zusammen mit X₁ und R₄ die oben definierten Bedeutungen haben und
X₃ = Wasserstoff oder -CO-R₄ ist, worin R₄ Wasserstoff, C₁₋₆-Alkyl, C₁₋₆-Halogenalkyl, C₃₋₆-cycloalkyl, Phenyl oder Phenyl-alkyl (C₁₋₆) ist, wobei der Phenylring substituiert sein kann durch ein oder zwei: Halogen,
C₁₋₃-Alkyl, C₁₋₃-Alkoxy oder Nitro, oder
R₄ zusammen mit X₂ die oben definierten Bedeutungen hat oder R₄ zusammen mit X₂ und X₁ die oben definierten Bedeutungen haben oder
X₃ zusammen mit X₂ die oben definierten Bedeutungen hat und
X₄ = -O-SO₂R₆ ist, worin R₆ = Methyl, Trifluormethyl, Phenyl oder p-Methylphenyl ist und
(ii) die D-threo-Verbindung der Formel (I) mit KF in einem Polyglykol fluoriert wird, um die D-threo-Verbindung der Formel 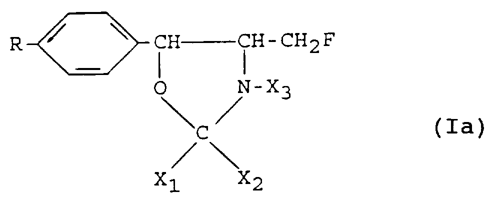 zu erhalten,
worin R, X₁, X₂ und X₃ die oben definierten Bedeutungen haben und
(iii) die D-threo-Verbindung der Formel (Ia) hydrolysiert wird, um eine D-threo-Verbindung der Formel 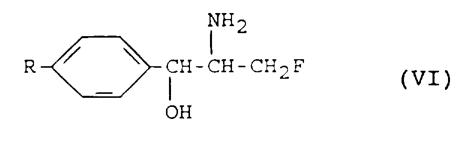 zu erhalten, worin R die oben definierten Bedeutungen hat und
(iv) die D-threo-Verbindung der Formel (VI) acyliert wird mit einer Verbindung der Formel R₁COOH, worin R₁ die oben definierte Bedeutung hat, oder ein reaktives Derivat davon zur Bildung der gewünschten D-threo-Verbindung der Formel (II).

## Revendications

1. Procédé de préparation d'un composé D-thréo de formule 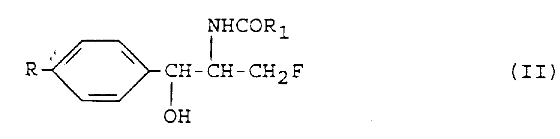 dans laquelle
R est un groupement méthylthio, méthylsulfoxy, méthylsulfonyle; et
R₁ est un groupement dichlorométhyle;
essentiellement dépourvu de sous-produits dérivant du remplacement du groupement hydroxy secondaire par un atome de fluor ou de la racémisation des atomes de carbone asymétriques,
caractérisé en ce que
(i) les groupements hydroxy secondaire et amino d'un D-thréo-1-(phényl)-2-amino-1,3-propanediol sont tous deux protégés via un composé D-thréo de formule 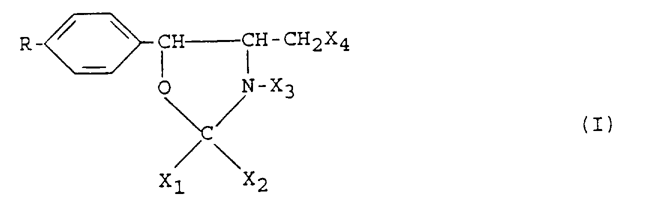 dans laquelle
R a les significations données ci-dessus;
X₁ est un atome d'hydrogène ou un groupement alkyle à 1-6 C, halogénoalkyle à 1-6 C, cycloalkyle à 3-6 C, phényle ou phénylalkyle (1-6 C) où le noyau phényle peut être substitué par un ou deux atomes d'halogène ou groupements alkyle à 1-3 C, alcoxy ou nitro; ou
X₁ avec X₂ est un atome d'oxygène ou un groupement alkylène ayant de deux à cinq atomes de carbone; ou
X₁ avec X₂ et R₄ est une chaîne de formule 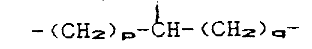 où p est 3 ou 4, et q est 1 ou 2; et
X₂ est un atome d'hydrogène ou un groupement alkyle à 1-6 C, halogénoalkyle à 1-6 C, cycloalkyle à 3-6 C ou phényle qui peut être substitué par un ou deux atomes d'halogène ou groupements alkyle à 1-3 C, alcoxy à 1-3 C ou nitro; ou
X₂ avec X₁ a les significations données ci-dessus; ou
X₂ avec X₃ est une liaison de covalence; ou
X₂ avec X₄ est
-(CH₂)ₘ-CH=CH-; -(CH₂)ₘ-CH₂-CH₂- 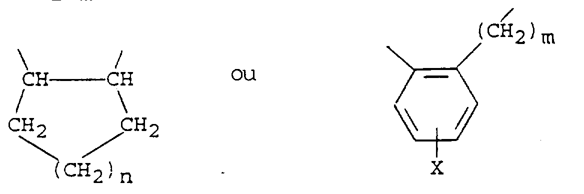
où n est 1 ou 2; m est 0 ou 1; X est un atome d'hydrogène ou d'halogène ou un groupement alkyle à 1-3 C, alcoxy à 1-3 C ou nitro; ou
X₂ avec X₁ et R₄ a les significations données ci-dessus; et
X₃ est un atome d'hydrogène ou -CO-R₄ où R₄ est un atome d'hydrogène ou un groupement alkyle à 1-6 C, halogénoalkyle à 1-6 C, cycloalkyle à 3-6 C, phényle ou phénylalkyle (1-6 C) où le noyau phényle peut être substitué par un ou deux atomes d'halogène ou groupements alkyle à 1-3 C, alcoxy à 1-3 C ou nitro, ou
R₄ avec X₂ a les significations données ci-dessus; ou
R₄ avec X₂ et X₁ a les significations données ci-dessus; ou
X₃ avec X₂ a les significations données ci-dessus; et
X₄ est -O-SO₂R₆ où R₆ est un groupement méthyle, trifluorométhyle, phényle ou p-méthylphényle; et
(ii) le composé D-thréo de formule (I) est fluoré par KF dans un polyglycol pour donner un composé D-thréo de formule 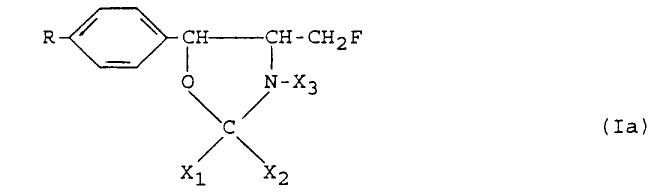 dans laquelle R, X₁, X₂ et X₃ ont les significations données ci-dessus: et
(iii) le composé D-thréo de formule (Ia) est hydrolysé pour fournir un composé D-thréo de formule 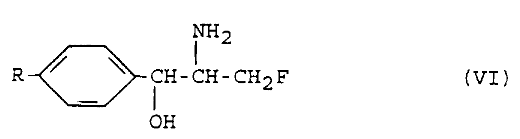 dans laquelle R a les significations données ci-dessus; et
(iv) le composé D-thréo de formule (VI) est acylé par un composé de formule R₁COOH où R₁ a les significations données ci-dessus, ou un de ses dérivés réactifs pour former le composé D-thréo de formule (II).
